# EUROPEAN PATENT SPECIFICATION

(11) **EP 2 130 489 B1**
(45) Date of publication and mention of the grant of the patent: **24.08.2016**
(21) Application number: 09161297.8
(22) Date of filing: 27.05.2009
(51) Int. Cl.: A61B 5/0476, A61B 5/055, G01R 33/563

(54) **Method for locating tracts of electrical brain activity**
Verfahren zur Ortung von Bereichen mit elektrischer Hirnaktivität
Procédé de localisation des voies de l'activité électrique du cerveau

(30) Priority: 06.06.2008 US 157068
(43) Date of publication of application: 09.12.2009
(60) Divisional application: 12163821.7
(73) Proprietor: Electrical Geodesics Inc., Eugene, OR 97403 (US)
(72) Inventor: Tucker, Don M., Eugene, OR 97405 (US)
(74) Representative: Fleuchaus, Michael A.

(56) References cited:
- EP-A2- 0 504 027
- WO-A-2004/051568
- WO-A-2007/124040
- US-A1- 2003 093 005
- K. HAMANDI ET AL.: "Combined EEG-fMRI and tractography to visualise propagation of epileptic activity" JOURNAL OF NEUROLOGY, NEUROSURGERY AND PSYCHIATRY, vol. 79, 20 December 2007 (2007-12-20), pages 594-597, XP002548643
- J. MATTOUT ET AL.: "MEG Source Localization under Multiple Constraints: An Extended Bayesian Framework" NEUROIMAGE, vol. 30, 20 December 2005 (2005-12-20), pages 753-767, XP002548644
- O. FAUGERAS ET AL.: "Variational, geometric, and statistical methods for modeling brain anatomy and function" NEUROIMAGE, vol. 23, 23 September 2004 (2004-09-23), pages S46-S55, XP002548645
- SYLVAIN BAILLET* ET AL: "A Bayesian Approach to Introducing Anatomo-Functional Priors in the EEG/MEG Inverse Problem", IEEE TRANSACTIONS ON BIOMEDICAL ENGINEERING, IEEE SERVICE CENTER, PISCATAWAY, NJ, USA, vol. 44, no. 5, 1 May 1997 (1997-05-01), XP011006368, ISSN: 0018-9294
- JBABDI S ET AL: "A Bayesian framework for global tractography", NEUROIMAGE, ACADEMIC PRESS, ORLANDO, FL, US, vol. 37, no. 1, 1 August 2007 (2007-08-01) , pages 116-129, XP026268025, ISSN: 1053-8119, DOI: 10.1016/J.NEUROIMAGE.2007.04.039 [retrieved on 2007-07-18]

## Description

### Field of the Invention

The present invention relates to a method for locating tracts of electrical brain activity, and more particularly relates to synergistic use of EEG and tractography for this purpose.

### Background of the Invention

For many purposes, such as evaluating the brain's health or understanding its operation, it is important to identify those portions of the brain which are, at a particular time or in response to a particular stimulus, active, as distinct from other portions of the brain which are not.

Brain activity is characterized by chemical reactions in brain cells, the chemical reactions generating electrical current and the electrical current being transmitted to other brain cells through nerve fibers, or "tracts" that establish a communication network. Therefore, characterizing the manner in which brain activity is integrated over regions of cortex could be described as determining the specific tracts that are active.

When a tract is active, i.e., it is conducting electricity or, more specifically, "neural currents." The neurons in the cortex that create, or that are affected by, those currents radiate electromagnetic energy. Electroencephalography (EEG) is the well known methodology by which this radiated energy is sensed, typically by electrodes placed on the surface of the head, and "source localization" is the methodology by which EEG data are processed for the purpose of discerning the location of the actively radiating cortical sources.

Source localization conceptually starts with a "forward problem" statement of the form y = Ax, wherein an independent source potential variable x produces an effect measured as the dependent measured potential variable y in accord with a transformation operator A. The source potential variable x is an ordered set corresponding to a number of discrete "sources" of electromagnetic radiation, and likewise the measure potential variable y is an ordered set corresponding to a number of sensors, typically electrodes placed on the surface of the body, at discrete locations. The transformation operator A is a matrix that specifies, generally, the physics of propagating electromagnetic radiation from points inside the body to points outside the body. The matrix A is determined generally by modeling, and may be determined by measurement as well. In any case, it is assumed to be known, and by use of the matrix operator A, a set of values y follows deductively from a given set of values x.

Source localization depends, however, on solving the inverse of the forward problem, i.e., the "inverse problem," which is to work backward from the sensed potentials to infer the set of source potentials that produced them.

The inverse problem is of the form A⁻¹y = x, which is to induce or infer the independent variable x from knowledge of the dependent variable y. There are a number of possible sets of circumstances x that are consistent with the observed results y, and identifying the correct set of circumstances requires additional criteria.

Such additional criteria are typically provided in the form of one or more assumptions regarding the expected behavior of the source variable x. A typical assumption is that the source variable x should exhibit spatial smoothness. For example, in what is known as the "minimum norm" approach, the source variable is constrained to minimum variance. Alternatively, Low Resolution Electromagnetic Tomographic Analysis (LORETA) minimizes the second derivative of the source variable in three dimensions, which smoothes the potential in three-space. Other criteria are described in S. BAILLET ET AL: "A Bayesian Approach to Introducing Anatomo-Functional Priors in the EEG/MEG Inverse Problem", IEEE Trans. on Biomed. Eng., vol. 44, no. 5, 1 May 1997, ISSN: 0018-9294.

Utilizing additional criteria allows selection of a "best" solution to the inverse problem in which the criteria are best met, and rejection of alternative solutions in which the criteria are less well met, or are not met. However, this strategy is effective only to the extent that the criteria are both accurate and capable of specifying the problem. Generally, neither is true, and there is a need for additional criteria for constraining solutions to the inverse problem.

While EEG is sensitive to brain activity, magnetic resonance imaging (MRI) is sensitive to brain structure, including the fiber tracts of the white matter. The imaging of tracts is known in the art as "tractography," and is typically accomplished by use of a special technique known as diffusion tensor imaging. Moreover, the tracts cannot be discerned absolutely; rather, they are discerned as being "likely" with an assigned probability. This is because diffusion tensor imaging merely indicates the existence of a tract within a given space in the brain and not its connectivity with other tracts, as described in S. JBABDI ET AL: "A Bayesian framework for global tractography",NEUROIMAGE, vol. 37, no. 1, 1 August 2007, pages 116-129, ISSN: 1053-8119.

In diffusion tensor imaging the brain is partitioned into a three-dimensional lattice having individual elements referred to in the computer arts as "voxels" (by analogy to the two-dimensional elements known as "pixels"). Each voxel has associated with it a number of "diffusion tensors" that indicate the direction of water diffusion. Since water diffuses along, rather than across, fiber tracts, tracts can be traced by chaining diffusion tensors of adjacent voxels.

The voxels establish a lower limit on the resolution of the capability of the MRI to image tracts. More particularly, it is common that more than one tract trace through the same voxel, and tractography does not have the resolution to establish which is which. Assume, for example, two tracts A and B that are traced through the same voxel. Tract A has an input end A1 that enters the voxel and an output end A2 that exits the voxel, and tract B has corresponding input and output ends B1 and B2. The MRI cannot "see" inside the voxel to discern whether A1 is connected to A2 or B2.

To partially mitigate this problem, a tractographic analysis provides a probabilistic assessment of the connectivity of A and B considering all voxels. For example, with regard to just the voxel C, the probability that A1 is connected to A2 is 0.5 or 50%.

Establishing overall probabilities for tract connectivity in consideration of how the tracts trace through all voxels is computationally more complicated, but the principle is the same. It is, however, a substantial drawback of the method that the result is probabilistic, not deterministic.

Accordingly, neither source localization nor tractography functions satisfactorily to identify the location of neural current sources or the continuity of nerve tracts, and so there is a need for improved methods and apparatus for locating tracts of electrical brain activity.

### Summary of the Invention

Improved methods for locating tracts of electrical brain activity are disclosed herein. According to one aspect of the invention, a representative method includes performing a first tractographic analysis of tracts in the brain, and performing an EEG procedure on the same brain. The EEG procedure includes virtually disposing a plurality of model sources in the brain and obtaining one or more solutions, corresponding to the model sources, to an inverse problem relating source and measured potentials. The inverse problem is solved at least in part by constraining the one or more solutions so as to take account of the first tractographic analysis.

Preferably, the method includes identifying a cortical surface of the brain, and virtually disposing the model sources on said cortical surface. The first tractographic analysis identifies ends of the tracts, and the method preferably further includes aligning the ends with the cortical surface and thereby identifying surface regions of the cortical surface associated with the aligned ends. Preferably, the method further includes associating the aligned ends with the model sources, wherein the first tractographic analysis indicates a first probability that at least two first sources of the model sources are connected by one or more first tracts, and wherein the solutions are constrained, at least in part, by constraining a covariance of said first sources.

According to an iterative aspect of the invention, the method may further include performing a second tractographic analysis indicating a second probability that at least two second sources of the model sources are connected by one or more second tracts, the second probability taking account of at least one of the solutions. Preferably, the second probability takes account of a covariance of the second sources established in at least one of the solutions.

According to another aspect of the invention, a representative method includes performing a first EEG procedure on the brain, including obtaining one or more first solutions to a first inverse problem relating source and measured potentials, and performing a tractographic analysis of the tracts, wherein the tractographic analysis indicates a first probability of the connectivity of at least two tracts taking account of the first solution.

Preferably, the method includes identifying a cortical surface of the brain, and virtually disposing first model sources on the cortical surface. The tractographic analysis identifies ends of the tracts, and the method preferably further includes aligning the ends with the cortical surface and thereby identifying surface regions of the cortical surface associated with the aligned ends. Preferably, the method still further includes associating the aligned ends with the first model sources, wherein the first EEG procedure includes indicating a covariance of at least two first sources of the first model sources, and the tractographic analysis takes account of the covariance in assessing a first probability that the first sources are connected by one or first more tracts.

According to another iterative aspect of the invention, the method may further include performing a second EEG procedure on the same brain including virtually disposing second model sources on the cortical surface and obtaining one or more second solutions to a second inverse problem relating source and measured potentials. The second solutions indicate that at least two second sources of the second model sources co-vary, and constraining the one or more second solutions so as to take account of the tractographic analysis.

Preferably, the one or more second solutions are constrained by a second probability that the second sources are connected by one or more second tracts established by the tractographic analysis.

It is to be understood that this summary is provided as a means of generally determining what follows in the drawings and detailed description and is not intended to limit the scope of the invention. Objects, features and advantages of the invention will be readily understood upon consideration of the following detailed description taken in conjunction with the accompanying drawings.

### Brief Description of the Drawings

Figure 1 is a schematic view of an EEG system for use according to the present invention.
Figure 2 is a schematic view of an MRI system for use according to the present invention.
Figure 3 is a flow diagram illustrating the use of the MRI system of Figure 2 to improve source localization provided by use of the EEG system of Figure 1 according to the present invention.
Figure 4 is a flow diagram illustrating the use of the EEG system of Figure 1 to improve tractography performed with the MRI system of Figure 2 according to the present invention.
Figure 5 is a flow diagram illustrating the use of the EEG and MRI systems of Figures 1 and 2 to improve both source localization and tractography according to the present invention, wherein a the results of a first tractography constrain the solutions to an inverse problem posed by an EEG procedure, and wherein the solutions to the inverse problem are taken into account in a second tractographic assessment of tract connectivity.
Figure 6 is a flow diagram illustrating the use of the EEG and MRI systems of Figures 1 and 2 to improve both source localization and tractography according to the present invention, wherein the results of a first EEG procedure are taken into account in a tractographic assessment of tract connectivity, and wherein the tractographic assessment of tract connectivity is utilized as a constraint in solving an inverse problem associated with a second EEG procedure.

### Detailed Description of Preferred Embodiments

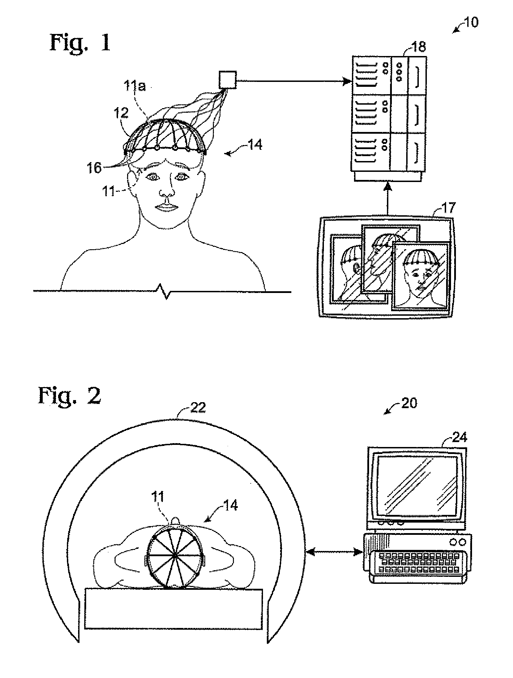

Referring to Figure 1, an EEG system 10 for performing an electroencephalographic procedure on a subject's brain 11 according to the present invention is shown. It should be understood that, while studying the brain is a preferred use of the invention and the invention is described herein in that particular context, the principles of the invention can be applied to the study of any internal body part that is electromagnetically or electrically active, such as the heart.

As is typical, the system includes a sensor net 12 that is fitted around the subject's head 14. The sensor net 12 includes, preferably, a large number of sensors 16, e.g., 256, for sensing the electromagnetic potentials produced by current flows through the tracts in the brain, otherwise known as neural currents. A larger number of sensors provides for greater measurement resolution and so it is desirable to provide as many sensors as is practical.

The sensors 16 are typically electrodes that make intimate contact with the skin; however any sensing elements capable of sensing electromagnetic fields or potentials, or related incidents such as current flows, disposed on or near the subject's head as appropriate, may be utilized.

It is important to know the location of the sensors on the head 14, and there are known means for accomplishing this. A preferred methodology utilizing a photogrammetric system 17 is described in U.S. Patent No. 7,190,826.

The sensors 16 are connected to a computer 18 that receives, digitizes, and processes the data from the sensors. The computer is also provided the sensor location information from the system 17.

Data are typically substantially continuously obtained over a time during which, for example, the subject thinks, rests, or sleeps. At each time instant however, particular neural currents are flowing inside the brain, producing particular electromagnetic potentials that are measured by the sensors corresponding to that time instant.

The inverse problem is solved at a particular time instant, though it typically need not be solved in real-time, so that it may be solved for a close succession of time instants to obtain an animated image of brain activity. In such cases, additional criteria for constraining solutions to the inverse problem may include requiring that the source potentials change smoothly over time as well as space. In any case, the inverse problem is solved according to the invention utilizing standard mathematical and computational techniques, subject to novel constraints.

One of these constraints is to (virtually) place sources utilized as the source potential variable x on the cortical surface. This requires identifying the cortical surface (11a in Figure 1), referred to herein as "cortical extraction." The present inventor has recognized that sources generally connect to the cortical surface and that a large number of the tracts also lie on this surface. Thus instead of modeling the brain as a three-dimensional volume, it is better to model the brain as a two-dimensional surface. Since the cortical surface is furrowed, it is often the case that two sources that are connected by a relatively long tract are spaced a relatively short distance from one another in three-dimensional space. For example, the two sources may be on opposite sides of a sulcus.

The invention may use standard techniques of cortical extraction, typically by use of standard MRI (magnetic resonance imaging) to obtain a three-dimensional image of the cortex. This defines a surface that is then tessellated in a computer (e.g., the computer 24 mentioned below) to create a mesh defining two-dimensional segments of the cortical surface referred to hereinafter as "patches." The patches define an area and a normal direction, i.e., perpendicular to the cortex. According to the invention, the brain model utilized in the EEG source localization is this tessellated cortical surface, whereon dipole sources are located at respective patches oriented along the associated normal directions.

A model source, preferably a dipole current source, is virtually disposed at a respective patch, and preferably each patch has associated with it a single dipole source. These sources define the aforementioned source variable x. The dipole sources are assumed to be connected to tracts, and it is recognized to be a good assumption that many of the dipole sources are connected by tracts to each other.

According to the invention, a tractographic analysis on the head 14 is carried out in the standard manner. Figure 2 shows a tractographic system 20 for use according to the present invention comprising an MRI scanner 22 and a computer 24 for controlling the scanner, receiving tomographic data indicating successive cross-sections of the brain 11, and producing a three-dimensional image of the brain. It should be understood that the computer utilized in the tractographic analysis may be the same computer (or computers) as the computer 18 mentioned above, or may be a different computer (or computers).

Within the resolution of the MRI scanner, the three-dimensional image specifies the geometry of various tissues in the head. Particularly, utilizing diffusion tensor imaging in the scanner 22, the computer 24 discerns an image of the tracts as well as produces a probabilistic assessment of their connectivity on a voxel-by-voxel basis.

The tract image locates the tracts and, therefore, the ends of the tracts. The tract image is aligned with the cortical image so that the ends of the tracts can be associated with particular patches on the cortical surface and, therefore, particular sources. This partially establishes how the sources may be connected. The probabilistic assessment further establishes how the sources may be connected. This allows an assessment of whether the sources should co-vary, i.e., whether activity at one source should correlate with activity at another.

Moreover, the tract image indicates the lengths of the tracts, and this information, in combination with the probabilistic assessment of tract connectivity, allows an assessment of the how the sources should co-vary, i.e., how the activity should be related in amplitude and/or time.

This is also a computationally complex determination, but it is not complex in principle, as can be appreciated by a simple example. Assume two tracts T1 and T2 the ends of which are determined to align with patches P1 and P2 corresponding to dipole sources D1 and D2. Based on a probabilistic assessment of tract connectivity, it is determined that there is a P% probability that the tracts T1 and T2 are connected in such manner as to establish a tract between the dipole sources D1 and D2 of length L. Given a known propagation velocity "PV" of an electrical signal carried by a tract, the length L corresponds to a time delay "TD," and so there is a P% chance that activity at the sources D1 and D2 will correlate with the time delay TD.

Similarly, if there is a known signal attenuation per unit of tract length, corresponding to an attenuation for the tract length L of "AT," then there is a P% chance that activity at the sources D1 and D2 will correlate with the attenuation AT.

Suppose it is more likely than not that the sources S1 and S2 co-vary (P > 50). In such case, it is more likely that limiting solutions to the inverse problem by requiring that S1 and S2 co-vary will be accurate than to fail to take this likely co-variance into account.

This is just one illustrative example, it being understood that there are numerous ways that additional information identifying and mapping, probabilistically or not, a tract network to the cortical surface, can be used to better specify the inverse problem.

It should be understood that two sources may be connected by any combination and number of parallel and serial tracts. Regardless of complexity, any such network can be analyzed as if it were an equivalent electrical circuit using well-known techniques. In such cases where a network of tracts defines an "electrical path" between two sources, a corresponding effective or overall "electrical path length" may be derived from such network analysis. For purposes herein, the terms "electrical path" and "electrical path length" are intended as generalizations of the terms "tract" and "tract length." More generally still, a network analysis defines a transfer function as is known in the electrical arts relating a signal at one source to a signal at the other.

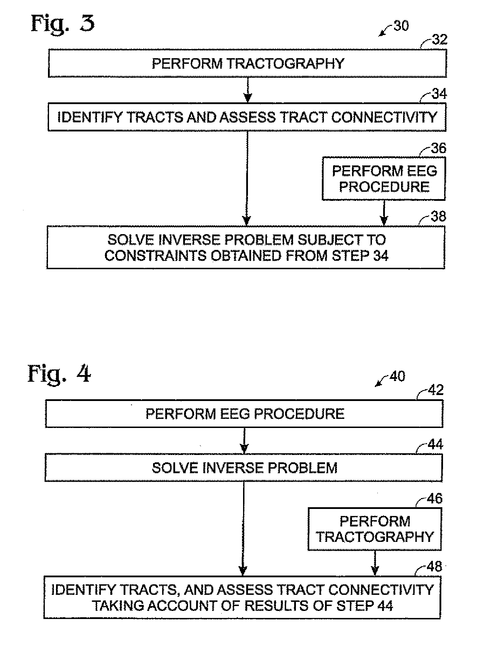

Figure 3 illustrates a methodology 30 of utilizing tractography to improve source localization. In a step 32, a standard tractography is performed; in step 34, tracts are identified and an assessment of tract connectivity is made; in step 36, an EEG procedure is performed, the timing of which need not bear any particular relationship to the timing of steps 32 and 34; and in step 38, the results of step 34 are utilized as constraints in solving the inverse problem posed by step 36.

Also according to the invention, solutions to the inverse problem posed by the EEG may be used to inform the probabilistic analysis in tractography. As indicated above, the tractographic assessment of connectivity is probabilistic because the resolution of the MRI is limited to a voxel, and a number of different tracts may be traced through a voxel. However, it is recognized that the EEG is very good at discerning source covariance, which is a by-product and therefore indicator of connectivity. Accordingly, even where the inverse problem is solved in the standard manner, the results define a source co-variance that usefully informs the probabilistic analysis.

More particularly, the source locations as they are estimated by one or more solutions to the inverse problem in the EEG may be aligned with the tract locations as imaged in the tractography. Thus, the tractography defines the tracts connecting the sources. If the sources co-vary, then the tracts connecting the sources must be connected.

As a simplified but illustrative example, assume two tracts determined by standard tractographic analysis to be connected with 50% probability. However, if the tracts were aligned with corresponding sources S1 and S2 that were determined by the EEG to co-vary, then the likelihood is clearly increased.

Moreover, if the correlation between the two sources indicates a delay time associated with a particular path length, the tract image may be utilized in conjunction with this information to identify likely voxel or voxels in which the tracts are connected.

For example, if the signal at S1 correlates well with the signal at S2 except that the signal at S1 is delayed relative to the signal at S2 by a time "t," then it can be assumed that the sources are connected by a network of tracts having an electrical path length PV • t. The tract image can be used to establish, probabilistically, a voxel, or set of voxels, corresponding to this path length.

Figure 4 illustrates a methodology 40 of utilizing source localization to improve tractography. In a step 42, a standard EEG procedure is performed; in step 34, the inverse problem is solved; in step 36, a tractography is performed, the timing of which need not bear any particular relationship to the timing of steps 42 and 44; and in step 48, the results of step 44 are taken into account in the assessment of tract connectivity.

In addition to utilizing standard tractographic assessments of tract connectivity to constrain solutions to the inverse problem in EEG, and utilizing solutions to the standard inverse problem in EEG to inform tractographic assessments of tract connectivity, it is further recognized that the two techniques can be utilized together such that each informs/constrains the other. As an example, assume a standard tractographic assessment of connectivity is made, and that the results are fed into the process of solving the inverse problem in a corresponding EEG as described above. Then the resulting solution(s) to the inverse problem is (are) fed into a re-evaluation of the probabilistic assessment of connectivity made from the original tractographic image. This provides both improved source localization and improved tractographic assessment of connectivity. The same result is obtained if the order is reversed (i.e., beginning with source localization instead of tractography).

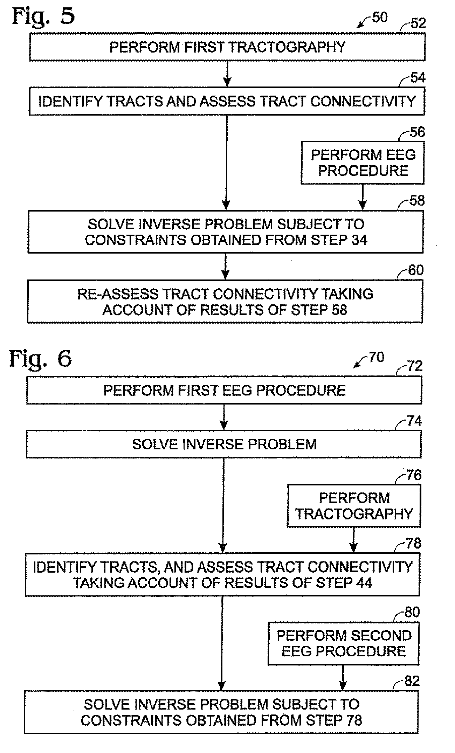

Figures 5 and 6 illustrate this collaborative methodology. In Figure 5, such a methodology 50 starts with steps 52 - 58, corresponding identically to steps 32 - 38 described above in connection with Figure 3. In step 60, are-assessment of tract connectivity is performed the timing of which need not bear any particular relationship to the timing of steps 52 - 56; and in step 60, the results of step 58 are taken into account. It may be noted that it is not necessary to perform a second imaging or identification of the tracts since the tract network will generally not have changed.

In Figure 6, such a methodology 70 starts with steps 72 - 78, corresponding identically to steps 42 - 48 described above in connection with Figure 4. In step 80, another EEG procedure is performed, the timing of which need not bear any particular relationship to the timing of steps 72 - 78; and in step 82, the results of step 78 are utilized as constraints in solving the inverse problem posed by step 80.

Further, according to the invention, the feedback process described above can be repeated any number of times to iterate toward further improved tractographic assessments and solution(s) to the inverse problem. Such iterations can provide improvements where the problem is under-specified, and in this case both the tractographic connectivity assessment problem and inverse problem are under-specified.

To appreciate this, assume an iteration in which there is a first tractography leading to an improved solution to an EEG source localization problem, and then the improved solution is utilized further to inform a second tractography. If the tractographic assessment is that tracts associated in the source localization with sources S1 and S2 are connected with 100% probability with a path length L, and the source localization therefore assumes that S1 and S2 co-vary with the corresponding time delay TD, constraining the connectivity of the same tracts in the second tractography to agree with this assessment renders the second tractography redundant with the first tractography.

However if, as a result of the first tractographic assessment, the source localization assumes that sources S1 and S2 should co-vary with time delay TD with a probability of only 75%, the uncertainty leaves room for the source localization to come to an uncertain conclusion that the sources S1 and S2 do not co-vary. This lack of certainty further leaves room for the second tractographic assessment to come to yet a different conclusion as to the probability that tracts associated with sources S1 and S2 are connected. This is more so for second tracts that are electrically distinct from the first tracts assumed in the source localization to be associated with the sources S1 and S2, where the first tractography assesses the second tracts an even less certain probability as to connectivity.

An analogous consideration applies for an iteration in which there is a first source localization leading to an improved tractographic analysis, and then the improved tractographic analysis is utilized further to constrain a second source localization. Due to the under-specificity of both the first source localization and the tractography, there is room for the second source localization to come to a different conclusion informed by both, the more so for sources S3 and S4 that are spatially distinct from the sources S1 and S2, where the uncertainty regarding the connectivity, and therefore the co-variance, of the sources S3 and S4 is greater than that regarding S1 and S2.

It is to be recognized that, while specific methods and apparatus for locating tracts of electrical brain activity have been shown and described as preferred, other configurations and methods could be utilized, in addition to configurations and methods already mentioned, without departing from the principles of the invention.

The terms and expressions which have been employed in the foregoing specification are used therein as terms of description and not of limitation, and there is no intention of the use of such terms and expressions to exclude equivalents of the features shown and described or portions thereof, it being recognized that the scope of the invention is defined and limited only by the claims which follow.

## Claims

1. A method for localizing electromagnetic sources in the brain, including
performing an EEG procedure (36, 56) on the brain (11) by an EEG system (10), including measuring electromagnetic potentials and virtually disposing a plurality of first model sources in the brain (11) and obtaining one or more first solutions, corresponding to said first model sources, to an inverse problem relating said first model sources and said measured potentials for localizing real sources of neural activity in the brain corresponding to said first model sources,
**characterized by**:
performing a first tractographic analysis (32,52) of one or more first tracts in the brain (11) by a tractographic system (20), and wherein said one or more first solutions are obtained at least in part by constraining said one or more solutions so as to take account of said first tractographic analysis.

2. The method of claim 1, the method further comprising identifying a cortical surface (11a) of the brain (11), and virtually disposing said first model sources on said cortical surface (11a).

3. The method of claim 2, the method further comprising tessellating said cortical surface (11a) so as to define a plurality of patches thereon, and disposing a single dipole source at each said patch.

4. The method of claim 2, wherein said first tractographic analysis (32,52) identifies ends of the tracts, the method further comprising aligning said ends with said cortical surface and thereby identifying surface regions of said cortical surface associated with the aligned said ends.

5. The method of claim 4, the method further comprising associating the aligned said ends with said first model sources, wherein said first tractographic analysis indicates a first probability that at least two first instances of said first model sources are connected by one or more first tracts, and wherein said step of constraining said one or more first solutions includes constraining a covariance of said first instances of said first model sources.

6. The method of claim 5, further comprising performing a second tractographic analysis indicating a second probability that at least two second instances of said model sources are connected by one or more second tracts, said second probability taking account of at least one of said one or more first solutions.

7. The method of claim 6, wherein said one or more second tracts are electrically distinct from said one or more first tracts.

8. The method of claim 6, wherein said second probability takes account of a covariance of said second instances of said first model sources established in at least one of said one or more first solutions.

9. The method of claim 1, wherein said first tractographic analysis indicates a first probability that at least two first instances of said first model sources are connected by one or more first tracts, and wherein said step of constraining said one or more first solutions includes constraining a covariance of said first instances of said first model sources.

10. The method of claim 9, further comprising performing a second tractographic analysis indicating a second probability that at least two second Instances of said first model sources are connected by one or more second tracts, said second probability taking account of at least one of said one or more solutions.

11. The method of claim 10, wherein said one or more second tracts are electrically distinct from said one or more first tracts.

12. The method of claim 10, wherein said second probability takes account of a covariance of said second sources established in at least one of said one or more first solutions.

13. A method for assessing connectivity of tracts in the brain (11), including
performing a first tractographic analysis (46, 76) of the tracts by a tractographic system (20), wherein said first tractographic analysis indicates a first probability of the connectivity of at least two tracts
**characterized by**
performing a first EEG procedure (42,72) on the brain (11) by an EEG system (10), including virtually disposing a plurality of first model sources in the brain, measuring electromagnetic potentials and obtaining one or more first solutions, corresponding to said first model sources, to a first EEG inverse problem relating said first model sources and said measured potentials for localizing real sources of neural activity in the brain corresponding to said first model sources; and
wherein the tractographic analysis is performed taking account of said one or more first solutions.

14. The method of claim 13, the method further comprising identifying a cortical surface (11a) of the brain, and virtually disposing said first model sources on said cortical surface (11a).

15. The method of claim 14, the method further comprising tessellating said cortical surface (11a) so as to define a plurality of patches thereon, and disposing a single one of said first model sources at each said patch.

16. The method of claim 14, wherein said first tractographic analysis (46, 76) identifies ends of the tracts, the method further comprising aligning said ends with said cortical surface (11a) and thereby identifying surface regions of said cortical surface (11a) associated with the aligned said ends.

17. The method of claim 16, the method further comprising associating the aligned said ends with said first model sources, wherein said first EEG procedure (42,72) indicates a covariance of at least two instances of said first model sources, and wherein said tractographic analysis (46,76) takes account of said covariance in assessing a first probability that said at least two instances of said first model sources are connected by one or more first tracts.

18. The method of claim 17, further comprising performing a second EEG procedure (80) on the brain (11), including virtually disposing second model sources on said cortical surface, and obtaining one or more second solutions, corresponding to said second model sources, to a second EEG Inverse problem relating said second model sources and measured potentials for localizing real sources of neural activity in the brain corresponding to said second model sources, indicating a covariance of at least two instances of said second model sources, and constraining said one or more second solutions so as to take account of said first tractographic analysis (46, 76).

19. The method of claim 18, wherein said at least two instances of said second model sources are spatially distinct from said at least two instances of said first model sources.

20. The method of claim 18, wherein said one or more second solutions are constrained by a second probability that said second model sources are connected by one or more second tracts established by said tractographic analysis (45, 76).

21. The method of claim 13, wherein said step of performing said first EEG procedure (42,72) includes indicating a covariance of at least two instances of said first model sources, and wherein said first tractographic analysis (46, 76) takes account of said covariance in assessing a first probability that said at least two instances of said first model sources are connected by one or more first tracts.

22. The method of claim 21, further comprising performing a second EEG procedure (80) on the brain (11), including virtually disposing a plurality of second model sources on said cortical surface and obtaining one or more second solutions, corresponding to said second model sources, to a second EEG Inverse problem relating said second model sources and measured potentials for localizing real sources of neural activity in the brain corresponding to said second model sources, indicating a covariance of at least two Instances of said second model sources, and constraining said one or more second solutions so as to take account of said first tractographic analysis (45, 76).

23. The method of claim 22, wherein said at least two instances of said second model sources are spatially distinct from said at least two instances of said first model sources.

24. The method of claim 22, wherein said one or more second solutions are constrained by a second probability that said second model sources are connected by one or more second tracts established by said first tractographic analysis (46, 76).

## Patentansprüche

1. Verfahren zum Lokalisieren von elektromagnetischen Quellen im Gehirn, enthaltend:
Durchführen einer EEG-Prozedur (36, 56) an dem Gehirn (11) durch ein EEG-System (10), einschließlich eines Messens von elektromagnetischen Potentialen und eines virtuellen Anordnens einer Mehrzahl von ersten Modellquellen in dem Gehirn (11) und eines Erhaltens von einer oder mehreren ersten Lösungen, die den ersten Modellquellen entsprechen, für ein inverses Problem, das die ersten Modellquellen und die gemessenen Potentiale in Beziehung setzt, um reale Quellen neuraler Aktivität in dem Gehirn, die den ersten Modellquellen entsprechen, zu lokalisieren, und
**gekennzeichnet durch**;
Durchführen einer ersten traktographlschen Analyse (32, 52) einer oder mehrerer erster Nervenbahnen In dem Gehirn (11) **durch** ein traktographisches System (20), und wobei die einen oder mehreren ersten Lösungen zumindest teilweise **durch** ein Eingrenzen der einen oder mehreren Lösungen erhalten werden, um die ersten traktographische Analyse zu berücksichtigen.

2. Verfahren gemäß Anspruch 1, wobei das Verfahren weiter umfasst, eine kortikale Oberfläche (11a) des Gehirns (11) zu identifizieren und die ersten Modellquellen virtuell auf der kortikalen Oberfläche (11a) anzuordnen.

3. Verfahren gemäß Anspruch 2, wobei das Verfahren weiter umfasst, die kortikale Oberfläche (11a) zu tessellieren, um eine Mehrzahl von Bereichen darauf zu definieren, und eine einzelne Dipolquelle an jedem der Bereichen anzuordnen.

4. Verfahren gemäß Anspruch 2, bei dem die erste traktographische Analyse (32, 52) Enden der Nervenbahnen identifiziert, wobei das Verfahren weiter umfasst, die Enden mit der kortikalen Oberfläche auszurichten und dadurch aberflächenregionen der kortikalen Oberfläche zu identifizieren, die mitden ausgerichteten Enden assoziiert sind.

5. Verfahren gemäß Anspruch 4, wobei das Verfahren weiter umfasst, die ausgerichteten Enden mit den ersten Modellquellen zu assozileren, wobei die erste traktographische Analyse eine erste Wahrscheinlichkeit dafür anzeigt, dass mindestens zwei erste Instanzen der ersten Modellquellen durch eine oder mehrere erste Nervenbahnen verbunden sind, und wobei der Schritt des Eingrenzens der einen oder mehreren ersten Lösungen ein Eingrenzen einer Kovarianz der ersten Instanzen der ersten Modeliquallen enthält.

6. Verfahren gemäß Anspruch 5, das weiter umfasst, eine zweite traktographische Analyse durchzuführen, die eine zweite Wahrscheinlichkeit dafür anzeigt, dass mindestens zwei zweite Instanzen der Modellquellen durch eine oder mehrere zweite Nervenbahnen verbunden sind, wobei die zweite Wahrscheinlichkeit mindestens eine der einen oder mehreren ersten Lösungen berücksichtigt.

7. Verfahren gemäß Anspruch 6, bei dem die eine oder mehreren zweiten Nervenbahnen elektrisch distinkt von den einen oder mehreren ersten Nervenbahnen sind.

8. Verfahren gemäß Anspruch 6, bei dem die zweite Wahrscheinlichkeit eine Kovarianz der zweiten Instanzen der ersten Modellquellen berücksichtigt, die durch mindestens eine der einen oder mehreren ersten Lösungen ermittelt wurden.

9. Verfahren gemäß Anspruch 1, bei dem die erste traktographische Analyse eine erste Wahrscheinlichkeit dafür anzeigt, dass mindestens zwei erste Instanzen der ersten Modellquellen durch eine oder mehrere erste Nervenbahnen verbunden sind, und wobei der Schritt des Eingrenzens der einen oder mehreren ersten Lösungen ein Eingrenzen einer Kovarianz der ersten Instanzen der ersten Modellquellen enthält.

10. Verfahren gemäß Anspruch 9, das weiter umfasst, eine zweite traktographische Analyse durchzuführen, die eine zweite Wahrscheinlichkeit dafür anzeigt, dass mindestens zwei zweite Instanzen derersten Modellquellen durch eine oder mehrere zweite Nervenbahnen verbunden sind, wobei die zweite Wahrscheinlichkeit mindestens eine der einen oder mehreren Lösungen berücksichtigt.

11. Verfahren gemäß Anspruch 10, bei dem die eine oder mehreren zweiten Nervenbahnen elektrisch distinkt von den einen oder mehreren ersten Nervenbahnen sind.

12. Verfahren gemäß Anspruch 10, bei dem die zweite Wahrscheinlichkeit eine Kovarianz der zweiten Quellen berücksichtigt, die durch mindestens eine der einen oder mehreren ersten Lösungen ermittelt wurden.

13. Verfahren zum Beurtellen der Konnektivität von Nervenbahnen im Gehirn (11), umfassend,
eine erste traktographische Analyse (46, 76) der Nervenbahnen durch ein traktographisches System (20) durchzuführen, wobei die erste traktographische Analyse eine erste Wahrscheinlichkeit der Konnektivität von mindestens zwei Nervenbahnen anzeigt,
**gekennzeichnet durch**:
Durchführen einer ersten EEG-Prozedur (42, 72) an dem Gehirn (11) **durch** ein EEG-System (10), einschließlich eines virtuellen Anordnens einer Mehrzahl von ersten Modellquellen in dem Gehirn, eines Messens von elektromagnetischen Potentialen und eines Erhaltens von einer oder mehreren ersten Lösungen, die den ersten Madellquellen entsprechen, für ein erstes EEG-Inversproblem, das die ersten Modellquellen und die gemessenen Potential in Beziehung setzt, um reale Quellen neuraler Aktivität in dem Gehirn, die den ersten Madellquellen entsprechen, zu lokalisieren; und
wobei die traktographische Analyse unter Berücksichtigung der einen oder mehreren ersten Lösungen durchgeführt wird.

14. Verfahren gemäß Anspruch 13, wobei das Verfahren weiter umfasst, eine kortikale Oberfläche (11a) des Gehirns zu identifizieren und die ersten Modellquellen virtuell auf der kortikalen Oberfläche (11a) anzuordnen.

15. Verfahren gemäß Anspruch 14, wobei das Verfahren weiter umfasst, die kartikale Oberfläche (11a) zu tessellieren, um eine Mehrzahl von Bereichen darauf zu definieren, und eine einzelne der ersten Modellquellen an jedem der Bereichen anzuordnen.

16. Verfahren gemäß Anspruch 14, bei dem die erste traktographische Analyse (46, 76) Enden der Nervenbahnen Identifiziert, wobei das Verfahren weiter umfasst, die Enden mit der kortikalen Oberfläche (11a) auszurichten und dadurch Oberflächen regionen der kortikalen Oberfläche (11a) zu identifizieren, die mit den ausgerichteten Enden assoziiert sind.

17. Verfahren gemäß Anspruch 16, wobei das Verfahren weiter umfasst, die ausgerichteten Enden mit den ersten Modellquellen zu assoziieren, wobei die erste EEG-Prozedur (42, 72) eine Kovarianz von mindestens zwei Instanzen der ersten Modellquellen anzeigt und wobei die traktographische Analyse (46, 76) die Kovarianz bei der Beurteilung einer ersten Wahrscheinlichkeit dafür berücksichtigt, dass die mindestens zwei Instanzen der ersten Modellquellen durch eine oder mehrere erste Nervenbahnen verbunden sind.

18. Verfahren gemäß Anspruch 17, das weiter umfasst, eine zweite EEG-Prozedur (80) an dem Gehirn (11) durchzuführen, einschließlich eines virtuellen Anordnens von zweiten Modellquellen auf der kortikalen Oberfläche und eines Erhaltensvon einer oder mehreren zweiten Lösungen, die den zweiten Modellquellen entsprechen, für ein zweites EEG-Inversproblem, das die zweiten Modellquellen und gemessenen Potentiale in Beziehung setzt, um reale Quellen neuraler Aktivität in dem Gehirn, die den zweiten Modellquellen entsprechen, zu lokalisieren, wodurch eine Kovarianz von mindestens zwei Instanzen der zweiten Madellquellen angezeigt wird, und die eine oder mehreren zweiten Lösungen einzugrenzen, um die erste traktographische Analyse (46, 76) zu berücksichtigen.

19. Verfahren gemäß Anspruch 18, bei dem die mindestens zwei Instanzen der zweiten Modellquellen räumlich distinkt von den mindestens zwei Instanzen der ersten Modellquellen sind.

20. Verfahren gemäß Anspruch 18, bei dem die eine oder mehreren zweiten Lösungen durch eine zweite Wahrscheinlichkeit dafür eingegrenzt werden, dass die zweiten modellquellen durch eine oder mehrere zweite Nervenbahnen verbunden sind, die durch die traktographische Analyse (46, 76) ermittelt wird.

21. Verfahren gemäß Anspruch 13, bei dem der Schritt der Durchführung der ersten EEG-Prozedur (42, 72) ein Anzeigen einer Kovarianz von mindestens zwei instanzen der ersten Modellquellen enthält und wobei die erste traktographische Analyse (46, 76) die Kovarianz bei der Beurteilung einer ersten Wahrscheinlichkeit dessen berücksichtigt, dass die mindestens zwei Instanzen der ersten Modellquellen durch eine oder mehrere erste Nervenbahnen verbunden sind.

22. Verfahren gemäß Anspruch 21, das weiter umfasst, eine zweite EEG-Prozedur (80) an dem Gehirn (11) durchzuführen, einschließlich eines virtuellen Anordnens einer Mehrzahl von zweiten Modellquellen auf der kortikalen Oberfläche und eines Erhaltens von einer oder mehreren zweiten Lösungen, die den zweiten Modellquellen entsprechen, für ein zweites EEG-Inversproblem, das die zweiten Modellquellen und gemessenen Potentiale In Beziehung setzt, um reale Quellen neuraler Aktivität in dem Gehirn, die den zweiten Modellquellen entsprechen, zu lokalisieren, wodurch eine Kovarianzvon mindestens zwei instanzen derzweiten Modellquellen angezeigt wird, und die eine oder mehreren zweiten Lösungen einzugrenzen, um die erste traktographlsche Analyse (46, 76) zu berücksichtigen.

23. Verfahren gemäß Anspruch 22, bei dem die mindestens zwei Instanzen derzweiten Modellquellen räumlich distinkt von den mindestens zwei instanzen der ersten Modellquellen sind.

24. Verfahren gemäß Anspruch 22, bei dem die eine oder mehrere zweiten Lösungen durch eine zweite Wahrscheinlichkeit dafür eingegrenzt werden, dass die zweiten Modellquellen durch eine oder mehrere zweite Nervenbahnen verbunden sind, die durch die erste traktographische Analyse (46, 76) ermittelt wird.

## Revendications

1. Méthode de localisation de sources électromagnétiques dans le cerveau, comportant la réalisation d'une première procédure d'EEG (36,56) sur le cerveau (11) par un système d'EEG (10), comportant la mesure de potentiels électromagnétiques et la disposition virtuelle d'une pluralité de premières sources de modèles dans le cerveau (11) ainsi que l'obtention d'une ou plusieurs premières solutions, correspondant auxdites premières sources de modèles, à un problème Inverse mettant en relation lesdites premières sources de modèles et lesdits potentiels mesurés en vue de la localisation des sources réelles d'activité neuronale dans le cerveau correspondant auxdites premières sources de modèles,
**caractérisée par**
la réalisation d'une première analyse tractographique (32, 52) d'un ou plusieurs tracts dans le cerveau (11) par un système tractographique (20), et dans laquelle lesdites une ou plusieurs premières solutions sont obtenues au moins en partie en contraignant une ou plusieurs solutions à prendre en compte ladite première analyse tractographique.

2. Méthode selon la revendication 1, la méthode comprenant par ailleurs l'identification d'une surface corticale (11a) du cerveau (11) et la disposition virtuelle desdites sources de modèles sur ladite source corticale (11a).

3. Méthode selon la revendication 2, la méthode comprenant par ailleurs la triangulation de ladite surface corticale (11a) afin de définir une pluralité de patchs sur celle-ci et la disposition d'une source de dipôle Individuelle sur chaque dit patch.

4. Méthode selon la revendication 2, dans laquelle ladite première analyse tractographique (32,52) identifie des extrémités des tracts, la méthode comprenant par ailleurs l'alignement desdites extrémités avec ladite surface corticale et ainsi l'identification des régions de surface de ladite surface corticale associée avec lesdites extrémités alignées.

5. Méthode selon la revendication 4, la méthode comprenant par ailleurs l'association desdites extrémités avec lesdites premières sources de modèles, dans laquelle ladite première analyse tractographique indique une première probabilité qu'au moins deux premiers exemples desdites premières sources de modèles soient reliés par un ou plusieurs premiers tracts et dans laquelle ladite étape de contrainte d'une ou plusieurs premières solutions comporte la contrainte d'une covariante desdits premiers exemples desdites premières sources de modèles.

6. Méthode selon la revendication 5, comprenant par ailleurs la réalisation d'une seconde analyse tractographique Indiquant une seconde probabilité qu'au moins deux seconds exemples desdites premières sources de modèles soient reliés par un ou plusieurs tracts, ladite seconde probabilité prenant en compte au moins une desdites une ou plusieurs premières solutions.

7. Méthode selon la revendication 6, dans laquelle lesdits un ou plusieurs seconds tracts sont distincts électriquement desdits un ou plusieurs premiers tracts.

8. Méthode selon la revendication 6, dans laquelle ladite seconde probabilité prend en compte une covariance desdits seconds exemples desdites premières sources de modèles établies dans au moins une desdites une ou plusieurs premières sortions.

9. Méthode selon la revendication 1, dans laquelle ladite première analyse tractographique indique une première probabilité qu'au moins deux premiers exemples desdites premières sources de modèles soient reliés par un ou plusieurs premiers tracts et dans laquelle ladite étape de contrainte de ladite une ou plusieurs premières solutions comporte la contrainte d'une covariante desdits premiers exemples desdites premières sources de modèles.

10. Méthode selon la revendication 9, comprenant par ailleurs la réalisation d'une seconde analyse tractographique indiquant une seconde probabilité qu'au moins deux seconds exemples desdites premières sources de modèles soient reliés par un ou plusieurs tracts, ladite seconde probabilité prenant en compte au moins une desdites une ou plusieurs premières solutions.

11. Méthode selon la revendication 10, dans laquelle lesdits un ou plusieurs seconds tracts sont distincts électriquement desdits un ou plusieurs premiers tracts.

12. Méthode selon la revendication 10, dans laquelle ladite seconde probabilité prend en compte une covariance desdites secondes sources établies dans au moins une desdites une ou plusieurs premières solutions.

13. Méthode d'évaluation de la connectivité des tracts dans le cerveau (11), comportant la réalisation d'une première analyse tractographique (46, 76) des tracts par un système tractographique (20), dans lequel ladite première analyse tractographique indique une première probabilité de la connectivité d'au moins deux tracts,
**caractérisée par**
la réalisation d'une première procédure d'EEG (42,72) sur le cerveau (11) par un système d'EEG (10), comportant la disposition virtuelle d'une pluralité de premières sources de
modèles dans le cerveau, la mesure de potentiels électromagnétiques et l'obtention d'une ou plusieurs premières solutions, correspondant auxdites premières sources de modèles, à un premier problème inverse d'EEG mettant en relation lesdites premières sources de modèles et lesdits potentiels mesurés en vue de la localisation des sources réelles d'activité neuronale dans le cerveau correspondant auxdites premières sources de modèles, et
dans laquelle l'analyse tractographique est réalisée en prenant en compte lesdites une ou plusieurs premières solutions.

14. Méthode selon la revendication 13, la méthode comprenant par ailleurs l'identification d'une surface corticale (11a) du cerveau et la disposition virtuelle desdites sources de modèles sur ladite surface corticale (11a).

15. Méthode selon la revendication 14, la méthode comprenant par ailleurs la triangulation de ladite surface corticale (11a) afin de définir une pluralité de patchs sur celle-ci et la disposition d'une Individuelle desdites sources de modèles sur chaque dit patch.

16. Méthode selon la revendication 14, dans laquelle ladite première analyse tractographique (46, 76) identifie des extrémités des tracts, la méthode comprenant par ailleurs l'alignement desdites extrémités avec ladite surface corticale (11a) et ainsi l'identlfication des régions de surface de ladite surface corticale (11a) associée avec lesdites extrémités alignées.

17. Méthode selon la revendication 16, la méthode comprenant par ailleurs l'association desdites extrémités alignées avec lesdites premières sources de modèle, dans laquelle ladite première procédure d'EEG (42, 72) indique une covariance d'au moins deux exemples desdites premières sources de modèles et dans laquelle ladite analyse
tractographique (46, 76) prend en compte ladite covariance en évaluant une première probabilité que lesdits au moins deux exemples desdites premières sources de modèles soient reliés par un ou plusieurs premiers tracts.

18. Méthode selon la revendication 17, comprenant par ailleurs la réalisation d'une seconde procédure d'EEG (80) sur le cerveau (11), comportant la disposition virtuelle de secondes sources de modèles sur ladite surface corticale, et l'obtention d'une ou plusieurs secondes solutions, correspondant auxdites secondes sources de modèles, à un second problème Inverse d'EEG mettant en relation lesdites secondes sources de modèles et lesdits potentiels mesurés en vue de la localisation des sources réelles d'activité neuronale dans le cerveau correspondant auxdites secondes sources de modèles, indiquant une covariance d'au moins deux exemples desdites secondes sources de modèles et la contrainte desdites une ou plusieurs secondes solutions à prendre en compte ladite première analyse tractographique (46, 76).

19. Méthode selon la revendication 18, dans laquelle lesdits au moins deux exemples desdites secondes sources de modèles sont distincts spatialement desdits au moins deux exemples desdites premières sources de modèles.

20. Méthode selon la revendication 18, dans laquelle lesdites une ou plusieurs secondes solutions sont contraintes par une seconde probabilité que lesdites secondes sources de modèles soient reliées par un ou plusieurs seconds tracts établis par ladite analyse tractographique (46, 76).

21. Méthode selon la revendication 13, dans laquelle ladite étape de réalisation de ladite première procédure d'EEG (42, 72) comporte l'indication d'une covariance d'au moins deux exemples desdites premières sources de modèles et dans laquelle ladite première analyse
tractographique (46, 76) prend en compte ladite covariance en évaluant une première probabilité que lesdits au moins deux exemples desdites premières sources de modèles soient reliés par un ou plusieurs premiers tracts.

22. Méthode selon la revendication 21, comprenant par ailleurs la réalisation d'une seconde procédure d'EEG (80) sur le cerveau (11), comportant la disposition virtuelle d'une pluralité de secondes sources de modèles sur ladite surface corticale et l'obtention d'une ou plusieurs secondes solutions, correspondant auxdites secondes sources de modèles, à un second problème inverse d'EEG en relation avec lesdites secondes sources de modèles et lesdits potentiels mesurés en vue de la localisation des sources réelles d'activité neuronale dans le cerveau correspondant auxdites secondes sources de modèles, indiquant une covariance d'au moins deux exemples desdites secondes sources de modèles et la contrainte desdites une ou plusieurs secondes solutions prendre en compte ladite première analyse tractographique (46, 76).

23. Méthode selon la revendication 22, dans laquelle lesdits au moins deux exemples desdites secondes sources de modèles sont distincts spatialement desdits au moins deux exemples desdites premières sources de modèles.

24. Méthode selon la revendication 22, dans laquelle lesdites une ou plusieurs secondes solutions sont contraintes par une seconde probabilité que lesdites secondes sources de modèles soient reliées par un ou plusieurs seconds tracts établis par ladite première analyse tractographique (46,76).
